## Europäisches Patentamt
## European Patent Office
## Office européen des brevets

(11) Veröffentlichungsnummer : **0 421 921 B1**

(12)

# EUROPÄISCHE PATENTSCHRIFT

(45) Veröffentlichungstag der Patentschrift :
**27.04.94 Patentblatt 94/17**

(51) Int. Cl.$^5$ : **A61K 31/66, A61K 9/54**

(21) Anmeldenummer : **90810661.0**

(22) Anmeldetag : **30.08.90**

(54) **Doppelt beschichtete Granulate.**

Die Akte enthält technische Angaben, die nach dem Eingang der Anmeldung eingereicht wurden und die nicht in dieser Patentschrift enthalten sind.

(30) Priorität : **07.09.89 CH 3245/89**

(43) Veröffentlichungstag der Anmeldung :
**10.04.91 Patentblatt 91/15**

(45) Bekanntmachung des Hinweises auf die Patenterteilung :
**27.04.94 Patentblatt 94/17**

(84) Benannte Vertragsstaaten :
**AT BE CH DE DK ES FR GB GR IT LI LU NL SE**

(56) Entgegenhaltungen :
**EP-A- 0 177 443
EP-A- 0 244 380
EP-A- 0 247 983
BRITISH MEDICAL JOURNAL, Band 295, 21.
November 1987, Seiten 1301-1305, London,
GB; H.I.J. HARINCK et al.
BR. J. CANCER, Band 61, Januar 1990, Seiten
123-125, Macmillan Press Ltd, London, GB;
D.J. DODWELL et al.**

(73) Patentinhaber : **CIBA-GEIGY AG
Klybeckstrasse 141
CH-4002 Basel (CH)**

(72) Erfinder : **Wirth, Dagmar, Dr.
Hauptstrasse 67A
CH-4312 Magden (CH)**
Erfinder : **Bucher, Christian
Rennweg 23
D-7800 Freiburg (DE)**

**Beschreibung**

Die Erfindung betrifft eine besonders vorteilhafte perorale Darreichungsform für 3-Amino-1-hydroxypropan-1,1-diphosponsäure-dinatriumsalz, Verfahren zur Herstellung dieser Darreichungsform sowie die Anwendung dieser Darreichungsform in einem therapeutischen Verfahren zur Behebung von Störungen des Calcium- und/oder Phosphatstoffwechsels.

3-Amino-1-hydroxypropan-1,1-diphosphonsäure und ihre Salze, ein Verfahren zur Herstellung dieser Säure sowie ihre technische Verwendung als Calciumkomplexbildender Waschmittelbestandteil ist in der Deutschen Auslegeschrift Nr. 2 130 794 beschrieben worden. Die Eignung der genannten Säure und ihrer Salze als pharmazeutischer Wirkstoff ist in der Deutschen Offenlegungsschrift Nr. 2 405 254 beschrieben worden. Das Dinatriumsalz - im folgenden Dinatriumpamidronat (engl. Freiname disodium pamidronate) genannt - ist als Antihyperkalzämikum bereits klinisch untersucht worden. Zahlreiche Publikationen belegen die gute Wirksamkeit dieser Verbindung gegen die besonders ernsten Krankheiten Osteoporose, Osteolyse infolge Metastasen in der Knochensubstanz und Pagetsche Krankheit.

Eine antihyperkalzämisch wirksame Verbindung muss sich ausserdem für die Langzeittherapie bis zu mehreren Monaten oder Jahren eignen. Für derart lange Anwendungszeiträume sind Darreichungsformen erforderlich, welche eine Verabreichung durch den Patienten ohne fremde Hilfe ermöglichen. Perorale Darreichungsformen wie Kapseln oder Tabletten sollten diesen Anforderungen entsprechen. Allerdings werden im British Medical J. Volume 295,1301-1305(1987), siehe Seite 1304, "epigastric complaints" bei klinischen Versuchen an Patienten nach Verabreichung von Kapseln oder Tabletten mit Dinatriumpamidronat erwähnt. Für orale Darreichungsformen mit verbesserter Magenverträglichkeit besteht daher ein Bedürfnis.

Für peroral verabreichte Granulate mit geringem Durchmesser, insbesondere Pellets mit einem Durchmesser von weniger als ca. 1,5 mm, ist deren beschleunigte Magenpassage charakteristisch. Diese können die von der Grösse der Inhaltsstoffe im Magen abhängige Sperrfunktion des Pylorus überwinden. Überzieht man die Granulate, insbesondere die Pellets, noch mit einer magensaftresistenten, darmsaftlöslichen Beschichtung, kann man eine trotz schnellerem Weitertransport noch mögliche Freisetzung des Wirkstoffs im Magen weitgehend eliminieren. Die Freisetzung findet dann gezielt im Duodenum statt, wo bessere Resorptionsverhältnisse gewährleistet sind. Der Wirkstoff wird in diesem Teil des Gastrointestinaltrakts schneller und auf grösserer Fläche als im Magen resorbiert, so dass hier das Risiko von lokalen Überkonzentrationen und Ulkusbildung gemindert ist.

Die Herstellung von Dinatriumpamidronat-Granulaten, welche mit einem magensaftresistenten, darmsaftlöslichen Beschichtungsmittel zu überziehen sind, ist problematisch, wenn man die üblichen Aufsprühverfahren anwendet. Die in solchen Verfahren benutzten organischen Lösungsmittel sind aus ökologischen Gründen ungünstig. Beim Aufsprühen von wässrigen Dispersionen auf die vorgeformten Granulate wird das Wasser in Kontakt mit dem alkalisch reagierenden Wirkstoff Dinatriumpamidronat basisch gemacht. Da das Beschichtungsmittel wegen der geforderten Löslichkeit im Dünndarmsaft im basischen Milieu löslich sein muss, sind Granulate mit diesem basisch reagierenden Wirkstoff für den Überzug mit einem solchen Beschichtungsmittel in Gegenwart von Wasser ungeeignet.

Der Erfindung liegt die Aufgabe zugrunde, Granulate von Dinatriumpamidronat, insbesondere in Form von Pellets, mit einem magensaftresistenten, darmsaftlöslichen Beschichtungsmittel so zu überziehen, dass dieses beim Aufsprühen mit einer wässrigen Dispersion des Beschichtungsmittels trotz Inkompatibilität mit dem basisch reagierenden Wirkstoff einen festen und gleichmässigen Ueberzug bildet.

Diese Aufgabe wird durch die vorliegende Erfindung gelöst, indem man die zu überziehenden Granulate, vorzugsweise Pellets, zunächst mit einer hydrophilen, elastischen inneren Beschichtung und anschliessend die so geschützten Granulate mit einer magensaftresistenten, darmsaftlöslichen äusseren Beschichtung überzieht.

In einer besonders bevorzugten Ausführungsform sind die doppelt beschichteten Granulate als sphärische Pellets mit einer Teilchengrösse kleiner als 1,5 mm geformt.

Die vorliegende Erfindung betrifft doppelt beschichtete Granulate für die perorale Applikation von Dinatriumpamidronat mit gesteuerter Freisetzung, dadurch gekennzeichnet, dass die Granulate mit einer hydrophilen, elastischen inneren Beschichtung und mit einer magensaftresistenten, darmsaftlöslichen äusseren Beschichtung überzogen sind.

Die doppelt beschichteten Granulate, insbesondere Pellets, zeichnen sich durch gute Magenverträglichkeit aus. Sie sind daher für die perorale Verabreichung in Kapseln und die Einnahme über längere Zeiträume hinweg besonders geeignet.

Die weiter vorn und im folgenden verwendeten Begriffe sind im Rahmen der Beschreibung der Erfindung wie folgt definiert:

In den doppelt beschichteten Granulaten gemäss der vorliegenden Erfindung ist der Wirkstoff Dinatriumpamidronat vorzugsweise in Form seines kristallinen Hydrats, insbesondere des Pentahydrats vorhanden, welches in der Europäischen Patentanmeldung Publikationsnr. 177 443 beschrieben ist.

Granulate sind feste Arzneimittelzubereitungen, welche den Wirkstoff Dinatriumpamidronat und solche Hilfsstoffe enthalten, die in der Pharmazeutischen Technologie für Tablettierverfahren üblich sind. Die Granulate gemäss vorliegender Erfindung sind selbst als perorale Darreichungsformen verwendbar, können aber auch zu Tabletten weiterverarbeitet werden.

Bevorzugt sind Granulate mit regelmässiger Formgebung, z.B. stäbchenförmig oder zylindrisch, insbesondere sphärisch, z.B. kugelförmig oder ellipsoid, welche man durch Extrusion der feuchten Granuliermasse durch Düsen, Ausrunden und Trocknen erhält.

Bevorzugt sind kugelförmige, sphärische Pellets mit einem Durchmesser zwischen ca. 0,3 und 1,5 mm, insbesondere zwischen ca. 0,5 und 1,25 mm.

Für die Herstellung der Granulate bzw. Pellets geeignete Hilfsstoffe sind z.B. pulverförmige Füllstoffe, z.B. mikrokristalline Cellulose, z.B. vom Typ Avicel® (FMC Corp.), z.B. der Typen AVICEL PH 101, 102, 105, RC 581 oder RC 591, Emcocel® (Mendell Corp.) oder Elcema® (Degussa), Kohlehydrate wie Zucker, Zuckeralkohole, Stärke oder Stärkederivate, z.B. Lactose, Dextrose, Saccharose, Glucose, Sorbit, Mannit, Xylitol, Kartoffel-, Mais-, Reis- oder Weizenstärke oder Amylopektin, Tricalciumphosphat, Calciumhydrogenphosphat oder Magnesiumtrisilicat, Bindemittel wie Celluloseäther, z.B. Methylcellulose, Carboxymethylcellulose oder Hydroxypropylmethylcellulose, Polyäthylenglycole bzw. Aethylenoxidhomopolymere, insbesondere mit einem Polymerisationsgrad von ca. $2,0 \times 10^3$ - $1,0 \times 10^5$ und einem ungefähren Molekulargewicht von ca. $1,0 \times 10^5$ - $5,0 \times 10^6$, z.B. unter der Bezeichnung Polyox® (Union Carbide) bekannte Kilfsstoffe, Polyvinylpyrrolidon bzw. Povidone, insbesondere mit einem mittleren Molekulargewicht von ca. 10000-360000, Polyvinylalkohol mit einem Hydrolysegrad von ca. 95-98 % und einem Polymerisationsgrad von ca. 500-2500, sowie Agar oder Gelatine, grenzflächenaktive Stoffe, z.B. anionische Tenside vom Typ Alkylsulfat, z.B. Natrium-, Kalium- oder Magnesium-n-dodecyl-sulfat, -n-tetradecyl-sulfat, -n-hexadecyl-sulfat oder -n-octadecyl-sulfat, Alkylethersulfat, z.B. Natrium-, Kalium- oder Magnesium-n-dodecyloxyethylsulfat, -n-tetradecyloxyethylsulfat, -n-hexadecyloxyethylsulfat oder -n-octadecyloxyethylsulfat oder Alkansulfonat, z.B. Natrium-, Kalium- oder Magnesium-n-dodecansulfonat, -n-tetradecansulfonat, -n-hexadecansulfonat oder -n-octadecansulfonat, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronics® (BWC) oder Synperonic® (ICI).

Der Begriff "perorale Applikation von Dinatriumpamidronat" definiert die Verwendbarkeit der doppelt beschichteten Granulate, vorzugsweise Pellets, als perorale Darreichungsformen, indem die doppelt beschichteten Granulate beispielsweise in Kapseln oder Sachets abgefüllt sind.

Kapseln sind vorzugsweise Steckkapseln aus Gelatine (Hartgelatine), welche gegebenenfalls unter Zusatz von Glycerin oder Sorbit hergestellt werden, sich durch Einwirken von Magensaft ohne zeitliche Verzögerung auflösen und dabei zunächst die doppelt beschichteten Granulate bzw. Pellets freisetzen. Diese werden durch den Pylorus in das Duodenum weitertransportiert. Geeignet sind, abhängig von der Dosierung, Steckkapseln der Grössen 0-4, vorzugsweise 0-2. Die Kapseln können transparent, farblos oder gefärbt sowie gegebenenfalls beschriftet sein, um diesen individuelles Aussehen bzw. sofortige Erkennbarkeit zu verleihen. Es eignet sich die Handelsware der Firmen Eli Lilly, Elanco, Capsugel oder Scherer.

Sachets sind Behältnisse, z.B. Beutel aus Polyäthylen, kaschiertem Papier oder Aluminium, welche die doppelt beschichteten Granulate enthalten. Nach dem Öffnen können die Granulate unmittelbar eingenommen werden.

Der Begriff "gesteuerte Freisetzung" definiert eine Beeinflussung der Freisetzung ("targeting") des Wirkstoffs Pamidronat-Dinatriumsalz, dass diese weitgehend nach der Magenpassage der Darreichungsform mit der Hauptmenge im Duodenum und gegebenenfalls mit einer Restmenge im Jejunum erfolgt.

Die hydrophile, elastische innere Beschichtung der doppelt beschichteten Granulate besteht aus einem filmähnlichen Material, das für Wasser bzw. Darmsaft durchlässig und in diesen Flüssigkeiten quellbar und zumindest teilweise löslich ist. Die innere Beschichtung verhindert die alkalische Reaktion des Wirkstoffs Dinatriumpamidronat mit der wässrigen Dispersion des magensaftresistenten, dünndarmsaftlöslichen Beschichtungsmittels.

Filmähnliche Materialien mit Wasserdurchlässigkeit sind z.B. hydrophile Gemische von Polyvinylpyrrolidon oder eines Copolymerisates von Polyvinylpyrrolidon und Polyvinylacetat mit Hydroxypropylmethylcellulose, Gemische von Schellack mit Hydroxypropylmethylcellulose, Polyvinylacetat oder dessen Copolymeren mit Polyvinylpyrrolidon, oder Gemische von wasserlöslichen Cellulosederivaten, wie Hydroxypropylmethylcellulose, und wasserunlöslicher Äthylcellulose.

3

EP 0 421 921 B1

Diese eigentlichen Beschichtungsmittel können, wenn erwünscht, im Gemisch mit anderen Kilfsstoffen, wie Talk oder Siliciumdioxid, z.B. synthetischer amorpher Kieselsäure vom Typ Syloid® (Grace), z.B. SYLOID 244 FP, oder Netzmitteln, z.B. den weiter vorn genannten Polyäthylenglycolen oder Sorbaten, verwendet werden.

Elastische, filmähnliche Materialien sind insbesondere hydrophile, partiell verätherte Cellulosederivate.

Hydrophile, partiell verätherte Cellulosederivate sind z.B. Niederalkyläther der Cellulose mit einem durchschnittlichen molaren Substitutionsgrad (MS) grösser als eins und kleiner als drei und einem durchschnittlichen Polymerisationsgrad von ca. 100-5000.

Der Substitutionsgrad ist ein Mass für die Substitution der Hydroxygruppen durch Niederalkoxygruppen pro Glucoseeinheit. Der durchschnittliche molare Substitutionsgrad (MS) ist ein gemittelter Wert und gibt die Anzahl der Niederalkoxygruppen pro Glucoseeinheit im Polymerisat an.

Der durchschnittliche Polymerisationsgrad (DP) ist ebenfalls ein gemittelter Wert und gibt die durchschnittliche Anzahl an Glucoseeinheiten im Cellulosepolymerisat an.

Niederalkyläther der Cellulose sind z.B. Cellulosederivate, die an der Hydroxymethylgruppe (primäre Hydroxygruppe) der die Celluloseketten bildenden Glucoseeinheit und gegebenenfalls an der zweiten und dritten sekundären Hydroxygruppe durch $C_1$-$C_4$-Alkylgruppen, insbesondere Methyl oder Aethyl, oder durch substituierte $C_1$-$C_4$-Alkylgruppen, z.B. 2-Hydroxyäthyl, 3-Hydroxy-n-propyl, Carboxymethyl oder 2-Carboxyäthyl, substituiert sind.

Geeignete Niederalkyläther der Cellulose sind vorzugsweise Cellulosederivate, die an der Hydroxymethylgruppe (primäre Hydroxygruppe) der Glucoseeinheit durch die genannten $C_1$-$C_4$-Alkyl- oder durch substituierte $C_1$-$C_4$-Alkylgruppen und an der zweiten und gegebenenfalls dritten sekundären Hydroxygruppe durch Methyl- oder Aethylgruppen substituiert sind.

Geeignete Niederalkyläther der Cellulose sind insbesondere Methylcellulose, Aethylcellulose, Methylhydroxyäthylcellulose, Methylhydroxypropylcellulose, Aethylhydroxyäthylcellulose, Hydroxyäthylcellulose, Hydroxypropylcellulose, Carboxymethylcellulose (in Salzform z.B. als Natriumsalz) oder Methylcarboxymethylcellulose (ebenfalls in Salzform z.B. als Natriumsalz).

Bevorzugte Niederalkyläther der Cellulose sind Methylcellulose (DP: ca. 200-1000, MS: ca. 1,4-2,0), Aethylcellulose (DP: ca. 150-1000, MS: ca. 1,2-1,8), z.B. vom Typ Aquacoat® (FMC Corp.), Hydroxyäthylcellulose (DP: ca. 120-1200, MS: ca. 1,2-2,5), Hydroxypropylcellulose (DP: ca 200-3000, MS: ca. 1,0-3,0) und Methylhydroxypropylcellulose (DP: ca. 200-1000, MS: ca. 1,4-2,0), z.B. vom Typ Pharmacoat® (Shin Etsu Corp.).

Die magensaftresistente, darmsaftlösliche äussere Beschichtung besteht aus einem filmähnlichen Material, das unter den sauren pH-Bedingungen des Magensafts für Wasser undurchlässig und unter den neutralen bis schwach basischen Bedingungen des Darmsafts, insbesondere im Duodenum, löslich ist.

Dieses filmähnliche Material kann auf die Granulate bzw. Pellets, die zuvor mit der hydrophilen, elastischen inneren Beschichtung versehen wurden, aus einer wässrigen Dispersion aufgetragen werden.

Filmähnliche Materialien mit Wasserlöslichkeit unter neutralen bis alkalischen Bedingungen sind z.B. Celluloseacetat-ester, z.B. Celluloseacetatphthalat (CAP), Celluloseacetattrimellitat (CAT) oder Methacrylsäure-Methacrylat- 1:1- oder 1:2-Copolymerisat, z.B. EUDRAGIT L und S, z.B. EUDRAGIT L 12.5 oder S 12.5.

Bevorzugt wird das filmähnliche Material als wässrige Dispersion von redispergierbarem Celluloseacetatphthalat - CAP - (Aquateric®: FMC), Polyvinylacetatphthalat - PVAP - (Coateric®: Colorcon), Hydroxypropylmethylcellulosephthalat - HPMCP (Aquacoat®: Shin-Etsu) sowie insbesondere partiell durch $C_1$-$C_4$-Alkylgruppen verestertem Acrylsäure-Methacrylsäure-Copolymerisat aufgesprüht.

Insbesondere wird ein partiell durch Methyl- und/oder Aethylgruppen verestertes Acrylsäure-Methacrylsäure- 1:1-Copolymerisat vom Typ EUDRAGIT L 30 D oder wässrig dispergiertes EUDRAGIT L 100-55 verwendet.

Das filmähnliche Material der äusseren magensaftresistenten, darmsaftlöslichen äusseren Beschichtung enthält bevorzugt zusätzliche Hilfsstoffe wie Weichmacher z.B. Triäthylcitrat, z.B. Citroflex® (Pfizer), Triacetin, diverse Phthalate, z.B. Diäthyl- oder Dibutylphthalat, gemischte Mono- oder Diglyceride vom Typ Myvacet® (Eastman), z.B. MYVACET 9-40, die weiter vorn genannten Polyäthylenglycole, z.B. mit Molmasse von ca. 6000-8000 sowie Aethylenoxid-Propylenoxid-Blockcopolymere vom Typ Pluronic® (BASF) oder Synperonic® (ICI), pulverförmige Trennmittel, z.B. Titandioxid, Talk, Magnesiumtrisilicat, Stärke oder synthetische, amorphe Kieselsäure vom Typ SYLOID, z.B.SYLOID 244FP.

Die wässrige Dispersion kann ausserdem zur Verhinderung der Schaumbildung grenzflächenaktive Stoffe wie ANTIFOAM AF enthalten.

Die Erfindung betrifft vorzugsweise Pellets des gemahlenen, kristallinen Pentahydrats, welche mit einer hydrophilen, elastischen inneren Beschichtung aus Hydroxypropylmethylcellulose und einer magensaftresistenten, darmsaftlöslichen äusseren Beschichtung eines partiell durch $C_1$-$C_4$-Alkylgruppen veresterten Acryl-

4

säure-Methacrylsäure- 1:1-Copolymerisat überzogen sind und bis zu 90 Gew.% Wirkstoffanteil enthalten.

Die Erfindung betrifft ebenfalls die Verwendung von Pamidronat-Dinatriumsalz, insbesondere des kristallinen Pentahydrats, zur Herstellung einer pharmazeutischen, festen Darreichungsform, vorzugsweise Kapseln oder Sachets, enthaltend doppelt beschichtete Granulate mit gesteuerter Freisetzung sowie diese Darreichungsformen selbst.

Die Herstellung von Granulaten mit Dinatriumpamidronat erfolgt in an sich bekannter Weise unter Anwendung von Verfahren zur Herstellung von Aufbau- oder Abbaugranulaten.

Verfahren zur Bildung von Aufbaugranulaten arbeiten kontinuierlich, z.B. durch gleichzeitiges Besprühen der Granuliermasse mit Granulierlösung und Trocknung, z.B. in der Granuliertrommel, in Granulierkesseln, auf Granuliertellern, im Fliessbett, durch Sprühtrocknung oder Sprüherstarrung oder diskontinuierlich, z.B. in der Wirbelschicht, im Chargenmischer oder in der Sprühtrocknungstrommel.

Bevorzugt sind Verfahren zur Herstellung von Abbaugranulaten, welche diskontinuierlich erfolgen können, indem die Granuliermasse mit der Granulierlösung zunächst ein feuchtes Aggregat bildet, das man anschliessend zu Granulaten, insbesondere Pellets, mit der gewünschten Körnung zerkleinert, wobei man bekannte Extrusions- und Sphäronisationsverfahren anwendet. Als Extruder und Ausrunder sind z.B. Geräte der Firmen Wyss & Probst, Werner & Pfleiderer, HKD, Loser, Fuji, Nica, Caleva u.a. geeignet.

Die Granuliermasse besteht aus dem zerkleinerten, vorzugsweise gemahlenen Wirkstoff Dinatriumpamidronat, vorzugsweise mit einer mittleren Teilchengrösse kleiner als 400 $\mu$m (mehr als 90 %) und den weiter vorn genannten Hilfsstoffen, z.B. pulverförmigen Füllstoffen wie mikrokristalliner Cellulose vom Typ AVICEL. Besonders geeignet ist AVICEL PH 105. Die Granuliermasse kann je nach angewendetem Verfahren vorgemischt oder durch Zumischen von ADP-Na$_2$ zu einem oder mehreren vorgelegten Hilfsstoffen oder durch Zumischen der Hilfsstoffe zu vorgelegtem Dinatriumpamidronat erhalten werden.

Bevorzugt stellt man Pellets mit sphäroider Formgebung und einer Korngrösse von ca. 0,5 bis 1,25 mm her.

Das Ueberziehen der Granulate bzw. der Pellets mit der hydrophilen, elastischen inneren Beschichtung erfolgt in an sich bekannter Weise unter Verwendung von üblichen Beschichtungsverfahren.

Beispielsweise wird das Beschichtungsmittel im gewünschten Mengenverhältnis in Wasser gelöst oder suspendiert. Gegebenenfalls setzt man Hilfsstoffe wie Polyäthylenglycol zu. Diese Lösung oder Dispersion wird auf die Granulate bzw. Pellets mit anderen Hilfsstoffen, z.B. Talk oder Siliciumdioxid, z.B. SYLOID 244 FP, aufgesprüht, z.B. unter Verwendung bekannter Verfahren, wie Sprühumhüllung in der Wirbelschicht z.B. in den Systemen von Aeromatic, Glatt, Wurster oder Hüttlin (Kugelcoater) sowie im Kessel nach den unter den Bezeichnungen Accela Cota oder Tauchrohrverfahren bekannten Verfahren.

Vorzugsweise wird eine wässrige Dispersion mit Hydroxypropylmethylcellulose (Cellulose HPM) aufgesprüht.

Das Ueberziehen der zuvor mit der hydrophilen, elastischen inneren Beschichtung beschichteten Granulate bzw. Pellets mit der magensaftresistenten, darmsaftlöslichen äusseren Beschichtung kann in an sich bekannter Weise nach denselben Verfahren erfolgen, welche man zuvor für das Ueberziehen der Granulate mit der inneren Beschichtung verwendet hat.

Bevorzugt wird ein Acrylsäure-Methacrylsäure-1:1-Copolymerisat vom Typ EUDRAGIT L 30 D als wässrige Dispersion in der Wirbelschicht unter Zusatz von Weichmachern wie Triäthylcitrat, Antischaummitteln und amorphem Siliciumdioxid, z.B. SYLOID 244 FP, aufgesprüht.

Die doppelt beschichteten Granulate bzw. Pellets werden anschliessend, gegebenenfalls unter Zusatz von Fliessreguliermitteln wie Talk oder amorphem Siliciumdioxid, in Kapseln, vorzugsweise Hartgelatinekapseln der Grösse 0, abgefüllt. Der Abfüllvorgang lässt sich mit einer der handelsüblichen Kapselabfüllmaschinen, z.B. Maschinen vom Typ Höfliger und Karg, Macofar oder MG2, durchführen. Die Kapseln lassen sich ihrerseits in Blisterpackungen oder Behältern aus Glas oder Polyäthylen abfüllen.

Die pharmazeutischen, festen Darreichungsformen der vorliegenden Erfindung zeichnen sich durch besonders gute gastrointestinale Verträglichkeit des Wirkstoffs Dinatriumpamidronat aus. Die Darreichungsformen sind für die Behandlung von Krankheiten geeignet, die mit Störungen des Calciumstoffwechsels in Verbindung gebracht werden können, beispielsweise entzündlichen Prozessen in Gelenken, degenerativen Prozessen im Gelenkknorpel, Osteoporosis, Periodontitis, Hyperparathyreoidismus und von Calciumablagerungen in Blutgefässen oder an prothetischen Implantaten. Günstig beeinflusst werden sowohl Erkrankungen, bei denen eine anomale Ablagerung schwerlöslicher Calciumsalze festzustellen ist, wie solcher aus den Formenkreisen der Arhritis, z.B. Morbus Bechterew, Neuritis, Bursitis, Periodontitis und Tendinitis, Fibrodysplasie, Osteoarthrose oder Arteriosklerose, als auch solche, bei denen eine anomale Auflösung harter Körpergewebe im Vordergrund steht, wie die hereditäre Hypophosphatasie, degenerative Prozesse im Gelenkknorpel, Osteoporosen verschiedener Genese, Morbus Paget und die Osteodystrophia fibrosa, ebenso durch Tumore ausgelöste osteolytische Prozesse sowie Hyperkalzämie.

Die folgenden Beispiele illustrieren die Erfindung. Dinatriumpamidronat abgekürzt: APD-Na$_2$.

Beispiel 1:

Kernpellet:

| | |
|---|---|
| APD-Na$_2$ Pentahydrat (gemahlen) | 197.3 mg |
| ($\hat{=}$ 150 mg wasserfreier Wirkstoff) | |
| Mikrokristalline Cellulose | 52.7 mg |
| (Avicel® PH 105) | |
| | 250.0 mg |

Innere Beschichtung:

| | |
|---|---|
| Cellulose-HP-M 603 | 10.0 mg |
| Polyäthylenglycol | 2.0 mg |
| Talk | 8.0 mg |
| | 270.0 mg |

Magensaftresistence äussere Beschichtung:

| | |
|---|---|
| Eudragit® L 30 D (Feststoff) | 90.0 mg |
| Triäthylcitrat | 21.0 mg |
| Antifoam® AF | 2.0 mg |
| Wasser | |
| Talk | 7.0 mg |
| | 390.0 mg |

Eine Mischung von APD-Na$_2$ mit Avicel® PH 105 wird mit Wasser befeuchtet und geknetet, extrudiert und sphäronisiert. Die getrockneten Pellets werden anschliessend in der Wirbelschicht nacheinander mit einer inneren Beschichtung, bestehend aus Cellulose-HP-M 603, Polyäthylenglycol (PAEG) 8000 und Talk, und dem wässrigen magensaftresistenten Lack, bestehend aus Eudragit® L 30 D, Triäthylcitrat und Antifoam® AF, beschichtet. Die lackierten Pellets werden mit Talk bepudert und mittels einer handelsüblichen Kapselabfüllmaschine z.B. Höfliger und Karg, in Kapseln (Kapselgrösse 0) abgefüllt.

Beispiel 2:

Kernpellet:

| | |
|---|---|
| APD-Na$_2$ Pentahydrat (gemahlen) | 197.3 mg |
| Mikrokristalline Cellulose | 52.7 mg |
| (Avicel® PH 105) | |
| | 250.0 mg |

Innere Beschichtung:

| | |
|---|---|
| Cellulose-HP-M 603 | 10.0 mg |
| PAEG 8000 | 2.0 mg |
| Talk | 8.0 mg |
| | 270.0 mg |

Magensaftresistence äussere Beschichtung:

| | |
|---|---|
| Eudragit L 30 D (Feststoff) | 90.0 mg |
| Triäthylcitrat | 21.0 mg |
| Antifoam® AF | 1.0 mg |
| Syloid® 244 FP | 9.0 mg |
| Wasser | |
| Talk | 4.0 mg |
| | 395.0 mg |

Man geht analog Beispiel 1 vor und fügt zur äusseren Beschichtung amorphes Siliciumdioxid SYLOID hinzu.

Beispiel 3:

Kernpellet:

| | |
|---|---|
| APD-Na$_2$ Pentahydrat/WS | 197.3 mg |
| Mikrokristalline Cellulose | 52.7 mg |
| (Avicel® PH 105) | |
| | 250.0 mg |

Innere Beschichtung:

| | |
|---|---|
| Cellulose-HP-M 603 | 10.0 mg |
| PAEG 8000 | 2.0 mg |
| Syloid$^{®}$ 244 FP | 8.0 mg |
| | ————— |
| | 270.0 mg |

Magensaftresistence äussere Beschichtung:

| | |
|---|---|
| Eudragit$^{®}$ L 30 D (Feststoff) | 90.0 mg |
| Triäthylcitrat | 21.0 mg |
| Antifoam$^{®}$ AF | 1.0 mg |
| Syloid$^{®}$ 244 FP | 9.0 mg |
| Wasser | |
| Talk | 4.0 mg |
| | ————— |
| | 395.0 mg |

Man verfährt analog Beispiel 1, ersetzt für die innere Beschichtung Talk durch SYLOID und fügt SYLOID ausserdem zur äusseren Beschichtung hinzu.

**Patentansprüche**

**Patentansprüche für folgende Vertragsstaaten : AT, BE, CH, DE, DK, FR, GB, IT, LI, LU, NL, SE**

1. Granulate für die perorale Applikation von Dinatrium-3-amino- 1-hydroxypropan-1,1-diphosphonat (Dinatriumpamidronat) mit doppelter Beschichtung und gesteuerter Freisetzung, dadurch gekennzeichnet, dass die Granulate mit einer hydrophilen, elastischen inneren Beschichtung und mit einer magensaftresistenten, darmsaftlöslichen äusseren Beschichtung überzogen sind.

2. Granulate gemäss Anspruch 1, dadurch gekennzeichnet, dass diese als sphärische Pellets geformt und mit einer hydrophilen, elastischen inneren Beschichtung und mit einer magensaftresistenten, darmsaftlöslichen äusseren Beschichtung überzogen sind.

3. Granulate gemäss Anspruch 2, dadurch gekennzeichnet, dass diese als sphärische Pellets mit einem Durchmesser von ca. 0,3 bis 1,5 mm geformt sind.

4. Granulate gemäss Anspruch 2, dadurch gekennzeichnet, dass diese als sphärische Pellets mit einem Durchmesser von ca. 0,5 bis 1,25 mm geformt sind.

5. Granulate gemäss einem der Ansprüche 1-4, dadurch gekennzeichnet, dass diese mit einer hydrophilen, elastischen inneren Beschichtung aus einem Celluloseäther überzogen sind.

6. Granulate gemäss einem der Ansprüche 1-4, dadurch gekennzeichnet, dass diese mit einer hydrophilen, elastischen inneren Beschichtung aus Hydroxypropylmethylcellulose überzogen sind.

7. Granulate gemäss einem der Ansprüche 1-6, dadurch gekennzeichnet, dass diese mit einer magensaftresistenten, darmsaftlöslichen äusseren Beschichtung eines partiell durch $C_1$-$C_4$-Alkylgruppen veresterten Acrylsäure-Methacrylsäure-Copolymerisats überzogen sind.

8. Granulate gemäss einem der Ansprüche 1-6, dadurch gekennzeichnet, dass diese mit einer magensaftresistenten, darmsaftlöslichen äusseren Beschichtung eines partiell durch Methyl- und/oder Äthylgruppen veresterten Acrylsäure-Methacrylsäure-Copolymerisats überzogen sind.

9. Pharmazeutische, feste Darreichungsform für die perorale Applikation des kristallinen Pentahydrats von Dinatriumpamidronat in Form von Kapseln oder Sachets enthaltend doppelt beschichtete Granulate mit gesteuerter Freisetzung, dadurch gekennzeichnet, dass die Granulate als sphärische Pellets geformt und mit einer hydrophilen, elastischen inneren und mit einer magensaftresistenten, darmsaftlöslichen äusseren Beschichtung überzogen sind.

10. Verfahren zur Herstellung von Granulaten gemäss Anspruch 1, dadurch gekennzeichnet, dass man Dinatriumpamidronat granuliert, die Granulate mit einer hydrophilen, elastischen inneren Beschichtung versieht und mit einer magensaftresistenten, darmsaftlöslichen äusseren Beschichtung überzieht.

11. Verfahren gemäss Anspruch 10, dadurch gekennzeichnet, dass man die Granulate als sphärische Pellets formt.

12. Verwendung von Dinatriumpamidronat zur Herstellung einer festen Darreichungsform in Form von Kapseln enthaltend doppelt beschichtete Granulate mit gesteuerter Freisetzung.

13. Verwendung des kristallinen Pentahydrats von Dinatriumpamidronat zur Herstellung einer festen Darreichungsform in Form von Kapseln enthaltend doppelt beschichtete Granulate mit gesteuerter Freisetzung.

14. Pharmazeutische, feste Darreichungsform gemäss Anspruch 9 zur Anwendung in einem therapeutischen Verfahren des menschlichen oder tierischen Körpers.

15. Pharmazeutische, feste Darreichungsform gemäss Anspruch 9 zur Anwendung in einem therapeutischen Verfahren bei der Behebung von Störungen des Calcium- und/oder Phosphatstoffwechsels.

**Patentansprüche für folgende Vertragsstaaten : ES, GR**

1. Verfahren zur Herstellung von Granulaten für die perorale Applikation von Dinatrium-3-amino-1-hydroxypropan-1,1-diphosphonat (Dinatriumpamidronat) mit doppelter Beschichtung und gesteuerter Freisetzung, dadurch gekennzeichnet, dass die Granulate mit einer hydrophilen, elastischen inneren Beschichtung und mit einer magensaftresistenten, darmsaftlöslichen äusseren Beschichtung überzogen sind.

2. Verfahren zur Herstellung von Granulaten gemäss Anspruch 1, dadurch gekennzeichnet, dass diese als sphärische Pellets geformt und mit einer hydrophilen, elastischen inneren Beschichtung und mit einer magensaftresistenten, darmsaftlöslichen äusseren Beschichtung überzogen sind.

3. Verfahren zur Herstellung von Granulaten gemäss Anspruch 2, dadurch gekennzeichnet, dass diese als sphärische Pellets mit einem Durchmesser von ca. 0,3 bis 1,5 mm geformt sind.

4. Verfahren zur Herstellung von Granulaten gemäss Anspruch 2, dadurch gekennzeichnet, dass diese als sphärische Pellets mit einem Durchmesser von ca. 0,5 bis 1,25 mm geformt sind.

5. Verfahren zur Herstellung von Granulaten gemäss einem der Ansprüche 1-4, dadurch gekennzeichnet, dass diese mit einer hydrophilen, elastischen inneren Beschichtung aus einem Celluloseäther überzogen sind.

6. Verfahren zur Herstellung von Granulaten gemäss einem der Ansprüche 1-4, dadurch gekennzeichnet, dass diese mit einer hydrophilen, elastischen inneren Beschichtung aus Hydroxypropylmethylcellulose überzogen sind.

7. Verfahren zur Herstellung von Granulaten gemäss einem der Ansprüche 1-6, dadurch gekennzeichnet, dass diese mit einer magensaftresistenten, darmsaftlöslichen äusseren Beschichtung eines partiell durch $C_1$-$C_4$-Alkylgruppen veresterten Acrylsäure-Methacrylsäure-Copolymerisats überzogen sind.

8. Verfahren zur Herstellung von Granulaten gemäss einem der Ansprüche 1-6, dadurch gekennzeichnet, dass diese mit einer magensaftresistenten, darmsaftlöslichen äusseren Beschichtung eines partiell durch Methyl- und/oder Äthylgruppen veresterten Acrylsäure-Methacrylsäure-Copolymerisats überzogen sind.

9. Verfahren zur Herstellung einer pharmazeutischen, festen Darreichungsform für die perorale Applikation des kristallinen Pentahydrats von Dinatriumpamidronat in Form von Kapseln oder Sachets enthaltend doppelt beschichtete Granulate mit gesteuerter Freisetzung, dadurch gekennzeichnet, dass die Granulate als sphärische Pellets geformt und mit einer hydrophilen, elastischen inneren und mit einer magensaftresistenten, darmsaftlöslichen äusseren Beschichtung überzogen sind.

10. Verfahren zur Herstellung von Granulaten gemäss Anspruch 1, dadurch gekennzeichnet, dass man Dinatriumpamidronat granuliert, die Granulate mit einer hydrophilen, elastischen inneren Beschichtung versieht und mit einer magensaftresistenten, darmsaftlöslichen äusseren Beschichtung überzieht.

11. Verfahren gemäss Anspruch 10, dadurch gekennzeichnet, dass man die Granulate als sphärische Pellets formt.

## Claims

### Claims for the following Contracting States : AT, BE, CH, DE, DK, FR, GB, IT, LI, LU, NL, SE

1. Granules for the oral administration of disodium 3-amino-1-hydroxypropane-1,1-diphosphonate (disodium pamidronate) having a double coating and controlled release, wherein the granules are coated with a hydrophilic, elastic inner coating and a gastric juice-resistant, intestinal juice-soluble outer coating.

2. Granules according to claim 1, wherein these are formed as spherical pellets and coated with a hydrophilic, elastic inner coating and a gastric juice-resistant, intestinal juice-soluble outer coating.

3. Granules according to claim 2, wherein these are formed as spherical pellets having a diameter of about 0.3 to 1.5 mm.

4. Granules according to claim 2, wherein these are formed as spherical pellets having a diameter of about 0.5 to 1.25 mm.

5. Granules according to any one of claims 1-4, wherein these are coated with a hydrophilic, elastic inner coating of a cellulose ether.

6. Granules according to any one of claims 1-4, wherein these are coated with a hydrophilic, elastic inner coating of hydroxypropylmethylcellulose.

7. Granules according to any one of claims 1-6, wherein these are coated with a gastric juice-resistant, intestinal juice-soluble outer coating of an acrylic acid-methacrylic acid copolymer which is partially esterified by $C_1$-$C_4$ alkyl groups.

8. Granules according to any one of claims 1-6, wherein these are coated with a gastric juice-resistant, intestinal juice-soluble outer coating of an acrylic acid-methacrylic acid copolymer which is partially esterified by methyl and/or ethyl groups.

9. A solid pharmaceutical administration form for the oral administration of the crystalline pentahydrate of disodium pamidronate in the form of capsules or sachets containing double-coated granules having controlled release, wherein the granules are formed as spherical pellets and coated with a hydrophilic, elastic inner coating and a gastric juice-resistant, intestinal juice-soluble outer coating.

10. A method for the production of granules according to claim 1, wherein disodium pamidronate is granulated, the granules are provided with a hydrophilic, elastic inner coating and coated with a gastric juice-resistant, intestinal juice-soluble outer coating.

11. A method according to claim 10, wherein the granules are formed as spherical pellets.

12. The use of disodium pamidronate for the production of a solid administration form in the form of capsules containing double-coated granules having controlled release.

**13.** The use of the crystalline pentahydrate of disodium pamidronate for the production of a solid administration form in the form of capsules containing double-coated granules having controlled release.

**14.** A solid pharmaceutical administration form according to claim 9 for use in a therapeutic method for the human or animal body.

**15.** A solid pharmaceutical administration form according to claim 9 for use in a therapeutic method for the elimination of disorders of calcium and/or phosphate metabolism.

**Claims for the following Contracting States : ES, GR**

**1.** A method for the production of granules for the oral administration of disodium 3-amino-1-hydroxypropane-1,1-diphosphonate (disodium pamidronate) having a double coating and controlled release, wherein the granules are coated with a hydrophilic, elastic inner coating and with a gastric juice-resistant, intestinal juice-soluble outer coating.

**2.** A method for the production of granules according to claim 1, wherein these are formed as spherical pellets and coated with a hydrophilic, elastic inner coating and with a gastric juice-resistant, intestinal juice-soluble outer coating.

**3.** A method for the production of granules according to claim 2, wherein these are formed as spherical pellets having a diameter of about 0.3 to 1.5 mm.

**4.** A method for the production of granules according to claim 2, wherein these are formed as spherical pellets having a diameter of about 0.5 to 1.25 mm.

**5.** A method for the production of granules according to any one of claims 1-4, wherein these are coated with a hydrophilic, elastic inner coating of a cellulose ether.

**6.** A method for the production of granules according to any one of claims 1-4, wherein these are coated with a hydrophilic, elastic inner coating of hydroxypropylmethylcellulose.

**7.** A method for the production of granules according to any one of claims 1-6, wherein these are coated with a gastric juice-resistant, intestinal juice-soluble outer coating of an acrylic acid-methacrylic acid copolymer which is partially esterified by $C_1$-$C_4$ alkyl groups.

**8.** A method for the production of granules according to any one of claims 1-6, wherein these are coated with a gastric juice-resistant, intestinal juice-soluble outer coating of an acrylic acid-methacrylic acid copolymer which is partially esterified by methyl and/or ethyl groups.

**9.** A method for the production of a solid pharmaceutical administration form for the oral administration of the crystalline pentahydrate of disodium pamidronate in the form of capsules or sachets containing double-coated granules having controlled release, wherein the granules are formed as spherical pellets and coated with a hydrophilic, elastic inner coating and with a gastric juice-resistant, intestinal juice-soluble outer coating.

**10.** A method for the production of granules according to claim 1, wherein disodium pamidronate is granulated, the granules are provided with a hydrophilic, elastic inner coating and coated with a gastric juice-resistant, intestinal juice-soluble outer coating.

**11.** A method according to claim 10, wherein the granules are formed as spherical pellets.

**Revendications**

**Revendications pour les Etats contractants suivants : AT, BE, CH, DE, DK, FR, GB, IT, LI, LU, NL, SE**

**1.** Granulés pour l'administration perorale du 3-amino-1-hydroxypropane-1,1-diphosphonate disodique (pamidronate disodique) comportant un revêtement double et à libération contrôlée, caractérisés en ce que les granulés sont revêtus d'une couche interne hydrophile, élastique et d'une couche externe résistant au suc gastrique, soluble dans le suc intestinal.

2. Granulés selon la revendication 1, caractérisés en ce que lesdits granulés sont mis sous forme de pellets sphériques et en ce qu'ils sont revêtus d'une couche interne hydrophile, élastique et d'une couche externe résistant au suc gastrique, soluble dans le suc intestinal.

3. Granulés selon la revendication 2, caractérisés en ce que lesdits granulés sont mis sous forme de pellets sphériques ayant un diamètre compris entre environ 0,3 et environ 1,5 mm.

4. Granulés selon la revendication 2, caractérisés en ce que lesdits granulés sont mis sous forme de pellets sphériques ayant un diamètre compris entre environ 0,5 et environ 1,25 mm.

5. Granulés selon l'une des revendications 1 à 4, caractérisés en ce que lesdits granulés sont revêtus d'une couche interne, hydrophile, élastique, constituée d'un éther cellulosique.

6. Granulés selon l'une des revendications 1 à 4, caractérisés en ce que lesdits granulés sont revêtus d'une couche interne hydrophile, élastique, constituée d'hydroxypropylméthylcellulose.

7. Granulés selon l'une des revendications 1 à 6, caractérisés en ce que lesdits granulés sont revêtus d'une couche externe d'un copolymérisat acide acrylique-acide méthacrylique partiellement estérifié par des groupes alkyles en $C_1$-$C_4$, résistant au suc gastrique, soluble dans le suc intestinal.

8. Granulés selon l'une des revendications 1 à 6, caractérisés en ce que lesdits granulés sont revêtus d'une couche externe d'un copolymérisat acide acrylique-acide méthacrylique partiellement estérifié par des groupes méthyles et/ou éthyles, résistant au suc gastrique, soluble dans le suc intestinal.

9. Forme d'administration solide, pharmaceutique, pour l'administration perorale du pentahydrate de pamidronate disodique cristallisé sous forme de capsules ou de sachets contenant des granulés comportant un revêtement double à libération contrôlée, caractérisée en ce que les granulés sont mis sous forme de pellets sphériques et en ce qu'ils sont revêtus d'une couche interne hydrophile, élastique et d'une couche externe résistant au suc gastrique, soluble dans le suc intestinal.

10. Procédé pour la préparation des granulés selon la revendication 1, caractérisé en ce que l'on granule le pamidronate disodique, en ce que l'on munit les granulés d'un revêtement interne, hydrophile, élastique et en ce que l'on revêt les granulés d'une couche externe, résistant au suc gastrique, soluble dans le suc intestinal.

11. Procédé selon la revendication 10, caractérisé en ce que l'on met les granulés sous forme de pellets sphériques.

12. Utilisation du pamidronate disodique pour la préparation d'une forme d'administration solide sous forme de capsules contenant des granulés comportant un revêtement double à libération contrôlée.

13. Utilisation du pentahydrate de pamidronate disodique cristallisé pour la préparation d'une forme d'administration solide sous forme de capsules contenant des granulés comportant un revêtement double à libération contrôlée.

14. Forme d'administration solide, pharmaceutique, selon la revendication 9, destinée à être utilisée dans un procédé thérapeutique du corps humain ou animal.

15. Forme d'administration solide, pharmaceutique, selon la revendication 9, destinée à être utilisée dans un procédé thérapeutique pour la suppression des troubles du métabolisme du calcium et/ou des phosphates.

**Revendications pour les Etats contractants suivants : ES, GR**

1. Procédé pour la préparation des granulés pour l'administration perorale du 3-amino-1-hydroxypropane-1,1-diphosphonate disodique (pamidronate disodique) comportant un revêtement double et à libération contrôlée, caractérisé en ce que les granulés sont revêtus d'une couche interne hydrophile, élastique et d'une couche externe résistant au suc gastrique, soluble dans le suc intestinal.

2. Procédé pour la préparation des granulés selon la revendication 1, caractérisé en ce que lesdits granulés sont mis sous forme de pellets sphériques et en ce qu'ils sont revêtus d'une couche interne hydrophile élastique et d'une couche externe résistant au suc gastrique, soluble dans le suc intestinal.

3. Procédé pour la préparation des granulés selon la revendication 2, caractérisé en ce que lesdits granulés sont mis sous forme de pellets sphériques ayant un diamètre compris entre environ 0,3 et environ 1,5 mm.

4. Procédé pour la préparation des granulés selon la revendication 2, caractérisé en ce que lesdits granulés sont mis sous forme de pellets sphériques ayant un diamètre compris entre environ 0,5 et environ 1,25 mm.

5. Procédé pour la préparation des granulés selon l'une des revendications 1 à 4, caractérisé en ce que lesdits granulés sont revêtus d'une couche interne hydrophile, élastique, constituée d'un éther cellulosique.

6. Procédé pour la préparation des granulés selon l'une des revendications 1 à 4, caractérisé en ce que lesdits granulés sont revêtus d'une couche interne hydrophile, élastique, constituée d'hydroxypropylméthylcellulose.

7. Procédé pour la préparation des granulés selon l'une des revendications 1 à 6, caractérisé en ce que lesdits granulés sont revêtus d'une couche externe d'un copolymérisat acide acrylique-acide méthacrylique partiellement estérifié par des groupes alkyles en $C_1$-$C_4$, résistant au suc gastrique, soluble dans le suc intestinal.

8. Procédé pour la préparation des granulés selon l'une des revendications 1 à 6, caractérisé en ce que lesdits granulés sont revêtus d'une couche externe d'un copolymérisat acide acrylique-acide méthacrylique partiellement estérifié par des groupes méthyles et/ou éthyles, résistant au suc gastrique, soluble dans le suc intestinal.

9. Procédé pour la préparation d'une forme d'administration solide, pharmaceutique, pour l'administration perorale du pentahydrate de pamidronate disodique cristallisé sous forme de capsules ou de sachets contenant des granulés comportant un revêtement double à libération contrôlée, caractérisé en ce que les granulés sont mis sous forme de pellets sphériques et en ce qu'ils sont revêtus d'une couche interne hydrophile, élastique et d'une couche externe résistant au suc gastrique, soluble dans le suc intestinal.

10. Procédé pour la préparation des granulés selon la revendication 1, caractérisé en ce que l'on granule le pamidronate disodique, en ce que l'on munit les granulés d'un revêtement interne, hydrophile, élastique et en ce que l'on revêt les granulés d'une couche externe, résistant au suc gastrique, soluble dans le suc intestinal.

11. Procédé selon la revendication 10, caractérisé en ce que l'on met les granulés sous forme de pellets sphériques.